(19) Europäisches Patentamt
European Patent Office
Office européen des brevets

(11) Numéro de publication : **0 077 723**
**B1**

(12) **FASCICULE DE BREVET EUROPÉEN**

(45) Date de publication du fascicule du brevet : **12.02.86**

(51) Int. Cl.[4] : **C 07 D221/28**, A 61 K 31/485

(21) Numéro de dépôt : **82401876.6**

(22) Date de dépôt : **13.10.82**

(54) **Composition pharmaceutique à action antagoniste périphérique des narcotiques.**

(30) Priorité : **19.10.81 FR 8119607**

(43) Date de publication de la demande :
**27.04.83 Bulletin 83/17**

(45) Mention de la délivrance du brevet :
**12.02.86 Bulletin 86/07**

(84) Etats contractants désignés :
**AT BE CH DE FR GB IT LI LU NL SE**

(56) Documents cités :
**US-A- 4 176 186**
**HELVETICA CHIMICA ACTA, vol. 39, fasciculus II, no. 49, 1956, pages 429-440, Bâle, CH.**
**Dragonetti et al., Life Sciences 33 Sup.I (1983) 477-480**

(73) Titulaire : **SANOFI, société anonyme**
**40, Avenue George V**
**F-75008 Paris (FR)**

(72) Inventeur : **Giudice, Antonina**
**Via Cenisio no 61**
**I-20154 Milan (IT)**
Inventeur : **Moulineau, Claude**
**Lotissement des Genêts 61 avenue des Machics**
**F-34100 Montpellier (FR)**
Inventeur : **Manara, Luciano**
**Via Novella no 2**
**I-15040 Pietra Marazzi Alessandria (IT)**
Inventeur : **Carminati, Paolo**
**Via Pacini 24**
**Milano (IT)**

(74) Mandataire : **Gillard, Marie-Louise**
**Cabinet Beau de Loménie 55, Rue d'Amsterdam**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 077 723 B1

Jouve, 18, rue St-Denis, 75001 Paris, France

**Description**

La présente invention concerne une composition pharmaceutique à action antagoniste périphérique des opiacés.

Plus particulièrement, la présente invention a pour objet une composition pharmaceutique sous forme d'unité posologique renfermant un haloallylate de lévallorphan de formule

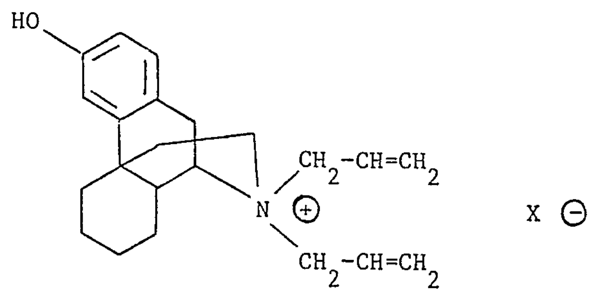

(I)

où X est chlore, brome ou iode, en mélange avec un excipient pharmaceutique.

Malgré les divers problèmes liés à l'usage des opiacés, tels que la tolérance et la dépendance physique, les médicaments morphiniques restent sans égaux dans le traitement de la douleur violente.

Un effet secondaire, souvent sérieux, des morphiniques est représenté par la constipation qui est due à leur action locale au niveau des récepteurs intestinaux des opiacés.

Pour pallier ce type d'effet secondaire très gênant pour le patient, il a été proposé qu'un antagoniste des narcotiques ayant une faible capacité de pénétration à travers la barrière hématoencéphalique pourrait réduire d'une façon significative la constipation provoquée par les médicaments morphiniques sans en réduire substantiellement les effets analgésiques.

Le brevet US 4 176 186 décrit des dérivés quaternaires d'antagonistes morphiniques qui préviennent ou éliminent les effets secondaires des opaciés sur la motilité intestinale. Parmi les composés revendiqués par le brevet ci-dessus, les composés préférentiels sont les dérivés quaternaires de la N-allylnoroxymorphone, ci-après désignée « naloxone », dont le bromométhylate est particulièrement préféré.

Hellerbach et al. dans Helvetica Chimica Acta *39,* fascicule II, n° 49 (1956) 420-440, décrit des antagonistes des opiacés, ayant une activité centrale et notamment le Levallorphan et son bromhydrate.

Il a été maintenant trouvé que les haloallylates de lévallorphan de formule I ci-dessus possèdent une capacité de pénétration de la barrière hématoencéphalique pratiquement nulle tout en conservant une bonne activité antagoniste périphérique.

Plus particulièrement, il a été trouvé que les haloallylates de lévallorphan de formule I ci-dessus n'antagonisent pas l'effet analgésique de la morphine tandis qu'ils antagonisent son effet constipant.

Le tableau I ci-dessous montre l'activité des halo-allylates de lévallorphan comme antagonistes des effets constipant et analgésique de la morphine en comparaison à l'action correspondante d'un antagoniste des narcotiques bien connu, la naloxone, ainsi que du sulfométhylate et du bromométhylate de naloxone décrits dans le brevet US 4 176 786.

L'antagonisme à l'effet constipant de la morphine a été évalué selon le test décrit par A. F. GREEN, Brit. J. Pharmacol. 1959, *14,* 26, opportunément modifié.

On utilise trois groupes de souris femelles de 20 grammes environ à jeun depuis 20 heures. A un groupe témoin, on administre par voie orale un repas constitué par 0,2 ml d'un mélange de gomme arabique 5 % (6 ml), farine (2 g) et charbon (1 g). Au deuxième groupe on administre par la voie sous-cutanée 12 mg/kg de morphine et, immédiatement après, le repas au charbon ci-dessus. Le troisième groupe est traité par l'antagoniste sous examen par la voie sous-cutanée, puis, après 5 minutes, par 12 mg/kg de morphine par voie sous-cutanée et, tout de suite après, par le repas au charbon. Trente minutes après, les animaux sont sacrifiés pour l'évaluation de la portion intestinale parcourue par le charbon, exprimée en % de la longueur totale de l'intestin grêle.

L'antagonisme à l'effet analgésique de la morphine a été évalué selon le test classique pour l'analgésie de l'électrostimulation de la queue chez la souris.

On utilise des souris femelles de 18 à 20 grammes. Les animaux, à jeun depuis 18 heures, sont placés dans de petites cages posées sur un plateau spécial, de façon à ce que les queues puissent sortir par un trou approprié qui a été pratiqué et se poser sur le plateau. Deux aiguilles sont enfilées dans la queue de chaque animal, la première à 1 cm de la racine de la queue et l'autre à 2 cm de la première aiguille. Les deux électrodes sont connectées à l'électrostimulateur Grass S 48 à l'aide duquel on cherche le voltage minimal qui, à la fréquence de 20 puls./sec. de la durée de 5 millisecondes et pendant 1 minute, détermine la réaction caractérisée par l'émission d'un cri de douleur. Après avoir établi le seuil de sensibilité, les animaux sont traités avec la substance à examiner et à intervalles pré-établis ils sont à nouveau stimulés pour la recherche du seuil nouveau. Un groupe d'animaux de contrôle est traité par de la morphine par

voie sous-cutanée à la dose de 12 mg/kg, qui détermine sur 100 % des animaux une augmentation moyenne de 100 % du seuil de sensibilité. Un autre groupe d'animaux est traité par la substance sous examen par voie sous-cutanée la morphine à la dose de 12 mg/kg. L'antagonisme à l'action analgésique de la morphine est évalué sur la base du pourcentage d'inhibition de l'effet analgésique de la morphine sur les animaux traités par rapport aux animaux de contrôle.

L'activité des substances à activité antagoniste périphérique des narcotiques est évaluée sur la base de l'inhibition pour cent de l'effet constipant et de l'effet analgésique de la morphine. La dose qui inhibe de 50 % les dits effets ($DI_{50}c$ pour l'effet constipant et $DI_{50}a$ pour l'effet analgésique) a été extrapolée de la droite de régression log dose-réponse en appliquant l'analyse de la variance par régression linéaire.

Tableau I

| Composé | Antagonisme à l'effet constipant | Antagonisme à l'effet analgésique | $DI_{50}a/DI_{50}c$ |
|---|---|---|---|
| | $DI_{50}c$ | $DI_{50}a$ | |
| lévallorphan chloroallylate | 27,30 mg/kg (12,00-62,00) | > 140 mg/kg(°) | > 5,12 (°) |
| lévallorphan bromoallylate | 15,65 mg/kg (8,46-28,9) | > 80 mg/kg(°) | > 5,11 (°) |
| lévallorphan iodoallylate | 16,68 mg/kg (8,23-33,80) | > 60 mg/kg(°) | > 3,61 (°) |
| naloxone bromométhylate | 10,81 mg/kg (6,2-18,8) | 5,117 mg/kg (1,3-19,7) | 0,47 |
| naloxone sulfométhylate | 2,99 mg/kg (1,51-5,92) | 5,039 mg/kg (1,8-14,0) | 1,68 |
| naloxone | 0,75 mg/kg (0,46-1,23) | 0,083 mg/kg (0,02-0,24) | 0,11 |

(°) inactif jusqu'à la dose maximale injectable

De ce tableau il ressort que les composés utilisés comme ingrédients actifs dans les compositions pharmaceutiques de la présente invention antagonisent l'effet constipant de la morphine avec une $DI_{50}c$ de 15,65 à 27,30 mg/kg alors que, jusqu'aux doses maximales injectables, ils n'antagonisent pas l'effet analgésique de la morphine. Par contre, les dérivés quaternaires de la naloxone décrits dans le brevet US 4 176 186, plus actifs des haloallylates de lévallorphan comme antagonistes de l'action constipante, antagonisent l'effet analgésique de la morphine avec une $DI_{50}a$ qui est assez proche de la $DI_{50}c$. De son côté, la naloxone est bien plus active comme antagoniste de l'effet analgésique que comme antagoniste de l'effet constipant.

Il en découle que les composés ingrédients actifs des compositions pharmaceutiques de la présente invention ne manifestent pas d'effets centraux jusqu'à la dose maximale injectable, montrant ainsi des antagonistes périphériques pratiquement purs, alors que, parmi le composés de référence, le bromométhylate et le sulfométhylate de nalaxone ont une certaine activité centrale et la naloxone elle-même est très active, se confirmant ainsi antagoniste central excellent.

Le bromoallylate de lévallorphan est décrit, avec ses constantes physico-chimiques, par J. Hellerbach, O. Schnider, H. Besendorf et B. Pellmont en Synthetic Analgesics, Editeurs D.H.R. Barton et W. Von Doering, Pergamon Press, 1974, part II, page 48, dans un tableau récapitulatif, mais aucune référence sur la synthèse chimique du produit ni sur ses propriétés pharmacologiques n'est indiquée par les Auteurs. Les mêmes Auteurs signalent, dans la référence n. 180 page 108 du même livre, que les données relatives au produit n'ont pas été rendues publiques. Les chloro- et iodoallytes sont nouveaux.

Les haloallylates de lévallorphan sont préparés par réaction d'un halogénure d'allyle, sur le lévallorphan dans un solvant organique à une température de 50 à 100 °C.

Comme solvant de réaction, on utilise un solvant polaire aprotique tel que la diméthylformamide, la diméthylacétamide, le diméthylsulfoxyde et similaires.

Le produit final est isolé selon les techniques habituelles en éliminant le solvant et en reprenant l'haloallylate de lévallorphan avec un solvant approprié.

Les compositions pharmaceutiques à action antagonistes périphérique des opiacés de la présente

invention sont utiles dans le traitement des conditions pathologiques où il existe des taux altérés d'opiacés exogènes ou endogènes, ou une hypersensibilité aux opiacés hors du système nerveux central des mammifères.

Ainsi, les compositions de la présente invention peuvent être administrées aux mammifères, animaux et êtres humains, avec les opiacés afin d'en prévenir les effets secondaires, en particulier la constipation, qui dérivent notamment de l'activation des récepteurs situés à la périphérie, sans compromettre l'analgésie, ou n'importe quelle autre action des opiacés, provoquée par stimulation des récepteurs centraux de la part de l'opiacé.

Afin d'obtenir l'effet antagoniste périphérique désiré, la dose de principe actif peut varier entre 0,05 et 200 mg par kg de poids du corps et par jour.

Chaque dose unitaire peut contenir de 1 à 500 mg d'ingrédient actif en combinaison avec un support pharmaceutique. Cette dose unitaire peut être administrée aux mammifères 1 à 4 fois par jour.

Les compositions pharmaceutiques de la présente invention à action antagoniste périphérique des opiacés peuvent être formulées par l'administration orale, sublinguale, sous-cutanée, intramusculaire, intraveineuse, transdermique ou rectale en mélangeant l'ingrédient actif de formule I ci-dessus avec des supports pharmaceutiques classiques.

Les formes unitaires d'administration comprennent les comprimés, les gélules, les poudres, les granules et les solutions ou suspensions orales, les suppositoires ainsi que les ampoules utiles pour une administration parentérale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de sucrose ou d'autres matières appropriées ou encore on peut les traiter d'une autre manière de telle sorte qu'ils aient une activité prolongée ou retardée et qu'ils libèrent continuellement une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir peut contenir l'ingrédient actif conjointement avec un édulcorant acalorique, du méthylparaben et du propylparaben comme antiseptiques, ainsi qu'un agent donnant du goût et un colorant approprié.

Les poudres ou les granulats dispersibles dans l'eau peuvent contenir l'ingrédient actif en mélange avec des agents de dispersion ou des agents mouillants, ou des agents de mise en suspension, tels que la polyvinylpyrrolidone et similaires, de même qu'avec des édulcorants ou des correcteurs du goût.

Pour une application rectale, on a recourt à des suppositoires qui sont préparés avec des liants fondant à la température rectale, par exemple du beurre de cacao ou des polyéthylèneglycols.

Pour une administration orale en gouttes ou pour une administration parentérale, on utilise des suspensions aqueuses, des solutions salines isotoniques ou des solutions particulières, stériles et injectables qui contiennent des agents de dispersion et/ou des mouillants pharmacologiquement compatibles, par exemple le propylèneglycol et le butylèneglycol.

Le principe actif peut être formulé également sous forme de microcapsules, éventuellement avec un ou plusieurs supports ou additifs.

Les compositions de la présente invention peuvent contenir, à côté de l'haloallylate de lévallorphan, d'autres principes actifs tels que, par exemple, des analgésiques narcotiques comme la morphine, la codéine, la buprénorphine et similaires, des antitussifs ou d'autres médicaments appropriés.

Les exemples suivants illustrent l'invention sans toutefois la limiter.

### Exemple 1

Un mélange de 1,2 g de lévallorphan, 20 ml de diméthylformamide anhydre et 0,98 g de bromure d'allyle est chauffé 4 heures à 70 °C à l'abri de l'humidité. Après élimination de la diméthylformamide, la gomme obtenue est triturée dans 30 ml d'acétone et, après 3 heures à 0 °C, le solide est essoré, lavé à l'acétone et séché sous pression réduite à 90 °C. On obtient ainsi 1,1 g de bromoallylate de lévallorphan ; p.f. 197-198 °C.

### Exemple 2

On prépare des comprimés ayant la composition suivante :

| | |
|---|---|
| bromoallylate de lévallorphan | 50 mg |
| lactose | 145 mg |
| avicel | 100 mg |
| stéarate de magnésium | 5 mg |

en broyant l'ingrédient actif jusqu'à une dimension des particules de 0,4 mm, en le faisant passer à travers un tamis de 0,4 mm d'ouverture de maille, en mélangeant la matière broyée avec les autres constituants et en comprimant pour former les comprimés.

De la même façon, on prépare des comprimés contenant 40 mg de bromoallylate de lévallorphan.

Exemple 3

On prépare des gélules à base de bromoallylate de lévallorphan ayant la composition suivante :

| | |
|---|---|
| bromoallylate de lévallorphan | 15 mg |
| lactose | 120 mg |
| stéarate de magnésium | 5 mg |

en mélangeant intimement les ingrédients ci-dessus et en versant le mélange dans des gélules de gélatine dure.

De la même façon, on prépare des gélules contenant 25 mg de bromoallylate de lévallorphan.

Exemple 4

10 000 gélules avec une teneur en substance active de 50 mg sont préparées à partir des constituants suivants : 500 g de bromoallylate de lévallorphan, 495 g de cellulose microcristalline, 5 g de gel de silice amorphe. Les constituants ci-dessus sont bien mélangés et on les introduit dans des gélules de gélatine dure de dimension 4.

Exemple 5

On prépare une solution aqueuse stérile appropriée pour une utilisation parentérale en ampoules ayant la composition suivante :

| | |
|---|---|
| bromoallylate de lévallorphan | 10 mg |
| eau pour préparation injectable | q.s.p. 2 ml |

Exemple 6

On prépare des suppositoires ayant la composition suivante :

| | |
|---|---|
| bromoallylate de lévallorphan | 50 mg |
| lactose | 250 mg |
| Witespol W 45 | q.s.p. 1,7 g |

On mélange la substance active avec le lactose et on met le mélange uniformément en suspension dans la masse pour suppositoires fondue. On verse la suspension dans des moules refroidis pour former des suppositoires d'un poids de 1,7 g.

Exemple 7

On prépare des comprimés dragéifiés contenant chacun 30 mg de bromoallylate de lévallorphan en utilisant comme excipient du talc, du lactose, de l'amidon de maïs, de l'alginate de sodium, du sucre semoule, du sucre cristallisé, du stéarate de magnésium, de la gomme laque blanche, de la gélatine alimentaire, de l'érythrosine, du bioxyde de titane et de la cire blanche.

Exemples 8 et 9

Par traitement du lévallorphan avec du chlorure d'allyle et, respectivement, de l'iodure d'allyle dans du diméthylformamide selon le mode opératoire décrit à l'Exemple 1, on obtient :
le chloroallylate de lévallorphan, solide blanc ayant un p.f. de 240 à 245 °C (déc) ; et, respectivement, l'iodoallylate de lévallorphan, solide brun ayant un p.f. de 225 à 230 °C (déc).

Exemples 10 à 15

En opérant comme décrit aux Exemples 2 à 7, on prépare :
— des comprimés renfermant 50 ou 40 mg de chloroallylate de lévallorphan,
— des gélules contenant 15 ou 25 mg de chloroallylate de lévallorphan,
— des gélules contenant 50 mg de chloroallylate de lévallorphan,
— des ampoules contenant 10 mg de chloroallylate de lévallorphan,
— des suppositoires renfermant 50 mg de chloroallylate de lévallorphan,
— des comprimés dragéifiés renfermant 30 mg de chloroallylate de lévallorphan.

Exemple 16

On prépare des comprimés dragéifiés contenant chacun 35 mg d'iodoallyle de lévallorphan en utilisant comme excipient du talc, du lactose, de l'amidon de maïs, de l'alginate de sodium, du sucre semoule, du sucre cristallisé, du stéarate de magnésium, de la gomme laque blanche, de la gélatine alimentaire, de l'érythrosine, du bioxyde de titane et de la cire blanche.

**Revendications** (pour les Etats contractants : BE, CH, DE, FR, GB, IT, LI, LU, NL, SE)

1. Un haloallylate de lévallorphan de formule

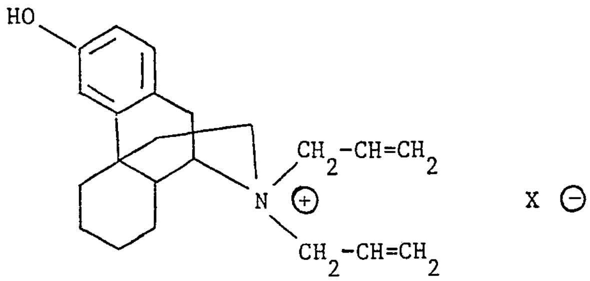

où X est chlore, brome ou iode, pour son application en tant que substance thérapeutiquement active.

2. Un haloallylate de lévallorphan selon la revendication 1 pour son application en tant que principe actif à action antagoniste périphérique des opiacés.

3. Une composition pharmaceutique sous forme d'unité posologique contenant un haloallylate de lévallorphan selon la revendication 1 et un excipient pharmaceutiquement inerte.

4. Une composition pharmaceutique selon la revendication 3 contenant de 1 à 500 mg d'haloallylate de lévallorphan.

5. Agent pour la prévention des effets secondaires dérivant de l'activation des récepteurs des opiacés situés à la périphérie, contenant un haloallylate de lévallorphan selon la revendication 1 et un excipient pharmaceutiquement inerte.

**Revendications** (pour l'Etat contractant AT)

1. Utilisation d'un haloallylate de lévallorphan de formule

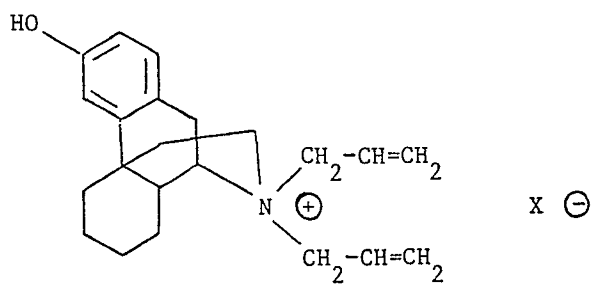

où X est chlore, brome ou iode, pour la fabrication d'une composition pharmaceutique à action antagoniste périphérique des opiacés.

2. Utilisation selon la revendication 1, caractérisée en ce que ledit haloallylate de lévallorphan est combiné avec excipient pharmaceutiquement inerte.

3. Utilisation selon l'une des revendications 1 ou 2, caractérisé en ce que ladite composition pharmaceutique contient de 1 à 500 mg d'haloallylate de lévallorphan.

**Claims** (for the Contracting States : BE, CH, DE, FR, GB, IT, LI, LU, NL, SE)

1. A levallorphan haloallylate of formula :

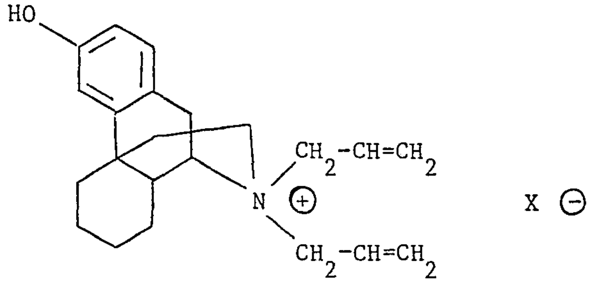

in which X is chlorine, bromine or iodine, for the application thereof as active therapeutic substance.

2. A levallorphan haloallylate according to claim 1, for the application thereof as active principle having peripheral antagonist activity on opiates.

3. A pharmaceutical composition in the form of a posological unit containing a levallorphan haloallylate according to claim 1, and a pharmaceutically inert excipient.

4. A pharmaceutical composition according to claim 3, containing 1 to 500 mg of levallorphan haloallylate.

5. Agent for preventing side effects resulting from the activation of opiate receptors situated at the periphery, containing a levallorphan haloallylate according to claim 1, and a pharmaceutically inert excipient.

**Claims** (for the Contracting State AT)

1. Use of a levallorphan haloallylate of formula :

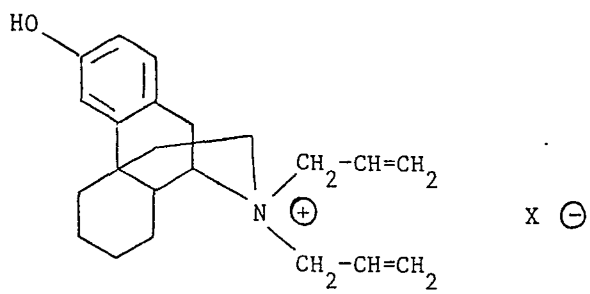

in which X is chlorine, bromine, or iodine, for preparing a pharmaceutical composition having peripheral antagonist activity on opiates.

2. Use according to claim 1, characterized in that said levallorphan haloallylate is used in combination with a pharmaceutically inert excipient.

3. Use according to any one of claims 1 or 2, characterized in that said pharmaceutical composition contains 1 to 500 mg of levallorphan haloallylate.

**Patentansprüche** (für die Vertragsstaaten : BE, CH, DE, FR, GB, IT, LI, LU, NL, SE)

1. Levallorphan-Halogenallylat der Formel

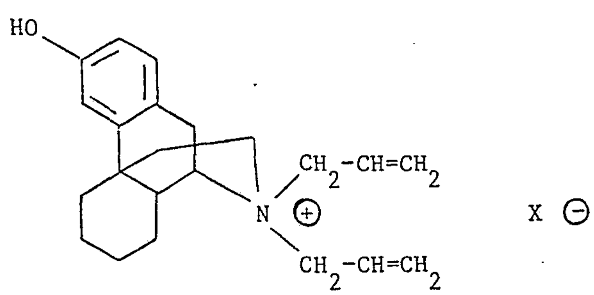

worin X für Chlor, Brom oder Jod steht, zur Anwendung als therapeutisch aktive Substanz.

2. Levallorphan-Halogenallylat nach Anspruch 1 zur Anwendung als Wirkstoff mit peripherer antagonistischer Wirkung zu Opiaten.

3. Pharmazeutische Zusammensetzung in Dosiseinheitsform, enthaltend ein Levallorphan-Halogenallylat nach Anspruch 1 und einen pharmazeutisch inerten Exzipienten.

4. Pharmazeutische Zusammensetzung nach Anspruch 3, enthaltend 1 bis 500 mg Levallorphan-Halogenallylat.

5. Mittel zum Verhindern von Nebenwirkungen bei der Aktivierung der an der Peripherie befindlichen Opiat-Rezeptoren, enthaltend ein Levallorphan-Halogenallylat nach Anspruch 1 und einen pharmazeutisch inerten Exzipienten.

**Patentansprüche** (für den Vertragsstaat AT)

1. Verwendung eines Levallorphan-Halogenallylats der Formel

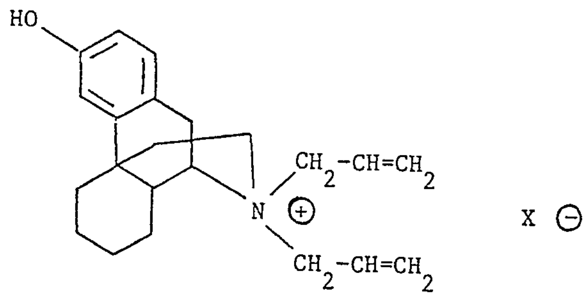

worin X für Chlor, Brom oder Jod steht, zur Herstellung einer pharmazeutischen Zusammensetzung mit peripherer antagonistischer Wirkung zu Opiaten.

2. Verwendung nach Anspruch 1, dadurch gekennzeichnet, daß das Levallorphan-Halogenallylat mit einem pharmazeutisch inerten Exzipienten kombiniert ist.

3. Verwendung nach einem der Ansprüche 1 oder 2, dadurch gekennzeichnet, daß die pharmazeutische Zusammensetzung 1 bis 500 mg Levallorphan-Halogenallylat enthält.